# EUROPEAN PATENT APPLICATION

(11) **EP 4 389 041 A1**
(43) Date of publication of application: **26.06.2024**
(21) Application number: 23216770.0
(22) Date of filing: 14.12.2023
(51) Int. Cl.: A61B 18/12

(54) **ELECTROSURGICAL GENERATOR HAVING A MODULAR OUTPUT SOCKET**

(30) Priority: 23.12.2022 US 202263434975 P
(71) Applicant: Olympus Winter & Ibe GmbH, 22045 Hamburg (DE)
(72) Inventor: Krüger, Jens, 15738 Zeuthen (DE); Dietrich, Stefan, 14469 Potsdam (DE); Stopp, Fabian, 10318 Berlin (DE)
(74) Representative: Glawe, Delfs, Moll

(57) **Abstract**

Electrosurgical generator configured to output a HF voltage to an electrosurgical instrument (9), comprising an inverter unit (24) generating the HF voltage supplied to an output socket (3), a control unit (1), and a user interface (4) for input/output. The inverter unit (24) and/or the output socket (3) is an exchangeable module comprising an independent memory device (6) having stored data sets on modes for usage of the respective module (5), and representation rules defining how operational data of the respective module shall be displayed. Once mounted in the electrosurgical generator, the data stored in the independent memory device can be accessed by the control unit and the user interface, thereby getting the information necessary for proper usage of the respective module and proper representation (44) of its particular functionality on the user interface (4). Thereby upgrading an existing generator with a later developed improved exchangeable module is facilitated.

## Description

The invention relates to an electrosurgical generator designed to output a high-frequency alternating voltage to an electrosurgical instrument. The electrosurgical generator comprises a main control unit and an inverter for high voltage that generates high-frequency voltage having a variable frequency and amplitude which is fed to an output socket for connection of the electrosurgical instrument.

In electrosurgery or high-frequency surgery, an electrosurgical instrument such as an electroscalpel is used to apply high-frequency alternating current to tissue in the human body. Usually high-frequency in the radiofrequency range of about 200 kHz to up to 4,000 kHz is used. This results in local heating of the tissue. Thereby, the tissue is cut or severed by heating, and the tissue is removed by thermal resection. A major advantage of this is that bleeding can be stopped at the same time as the cut by closing the affected vessels, and electrosurgical instruments can be used for other applications, such as coagulation.

In order to provide electrosurgical generators for various fields of medicine, different types of electrosurgical generators are required having different components and featuring different functionality. They are used for a variety of tasks in different fields of surgery, like urology, gynecology, gastroenterology, pulmonology, ENT (ear, nose, throat), phlebology and visceral surgery just to name a few. Functions of the electrosurgical generator differ depending on the medical field and the task at hand. Particularly, these functions differ in the way they provide power to the electrosurgical instrument. The different ways to supply the electrosurgical instrument with power are typically called "modes" by the persons skilled in the art. If just cutting of tissue is to be accomplished, then a cutting mode is selected providing a medium voltage continuously to the electrosurgical instrument. If coagulation is more important, then a different mode is selected, e.g., a spray or coagulation mode which uses rather high voltage with a low duty cycle. There are many more different modes for other kinds of applications.

For this reason, depending on the functionality desired, different kinds of electrosurgical instruments are provided that require i.a. different output sockets. For example, there are monopolar and bipolar instruments requiring monopolar and bipolar output sockets. Moreover, there are also universal output sockets being specially configured as to accept monopolar as well as bipolar electrosurgical instruments.

All this is managed by a main control unit of the respective electrosurgical generator. It controls actual operation of the electrosurgical generator including high-frequency voltage applied to the output sockets and the electrosurgical instruments and manages the user interface showing the necessary data to the user and processes user input. For ease of operation by the user and to avoid confusion about the function used and what is actually supplied by the output socket to the instrument, it is common to use different styles of presentation depending on the actual function used in the output socket employed. Accordingly, a visual, acoustical and functional representation of a standard "CUT" mode is yellow-themed and of a standard "COAG" (coagulation) mode is blue-themed, while other modes are the themed differently.

This is a concerted system having the advantage of providing the user a perceptive feedback. However, a concerted system is also a closed system which is difficult to expand or to upgrade.

It is an object of the invention to provide an improved electrosurgical generator which can be provided easier with expanded and upgraded output sockets and/or inverter units.

The solution according to the invention is found in the features of the independent claim. Advantageous developments are the subject matter of the dependent claims.

In an electrosurgical generator configured to output a high-frequency alternating voltage to an electrosurgical instrument, comprising a power supply unit, an inverter unit generating a high-frequency voltage which is output to at least one output socket for connection of the electrosurgical instrument, a main control unit configured to control operation of the electrosurgical generator, and a user interface for user input/output being functionally connected to the control unit, it is provided according to the invention that the inverter unit and/or the output socket is configured as exchangeable module, wherein the module comprises a power section for the high-frequency voltage, and a data section comprising an independent memory device having stored data sets on (i) modes relating to usage of the respective module, and (ii) representation rules defining how operational data of the respective module is to be displayed on the user interface, wherein the independent memory device is operationally connected to the control unit.

A few terms should first be explained below:
The inverter unit is a device to provide the actual high-frequency voltage output for the surgical instrument to be connected to the output socket. The term inverter is rather broad and comprises actual inverter technology as well as converters and amplifiers.

In the context of the present application the term "high-frequency" relates to frequencies in the radiofrequency range of 200 kHz to 4000 kHz as generated by the inverter of the electrosurgical generator. However, for electrosurgical generators being capable of driving ultrasound instruments, the term "high-frequency" also relates to the ultrasound frequency range, typically in the range of 20 kHz to 200 kHz (ultrasound surgical generators). The high frequency voltage considered as high voltage in the context of the present patent can have amplitudes in the high voltage range, in particular up to 10 kV, preferably up to 4000 Volt and further preferably more than 100 Volt.

The core of the invention is to configure the output socket and/or the inverter unit as an exchangeable module which has two specific sections. A "power section" which performs the core functionality of the respective module, i.e. generating high voltage in case of the inverter unit or supplying the generated voltage in a proper manner to the electrosurgical instrument in case of the output socket, and a "data section" which has an own memory device integrated into the respective module and having stored the data that is necessary for operating the module by the electrosurgical generator and for proper representation on the user interface of the electrosurgical generator. Once such a module is mounted in the electrosurgical generator, the data and information stored in the independent memory device can be accessed by the electrosurgical generator, in particular its main control unit and the user interface. To this end, the module with the independent memory device is configured to transfer the stored data sets to the main control unit which comprises a receiver configured to retrieve said stored data sets. Thereby, the main control unit and user interface gets information and data necessary for proper usage of the respective module and for proper representation of said respective module on the user interface, in particular the display, of the electrosurgical instrument.

Accordingly, the exchangeable modules for the output socket and the inverter unit bring with them all the information which is needed by the main control unit and the user interface for making proper and full use of said exchangeable module. Providing of new and improved exchangeable modules becomes thus independent from the functions and functionality as already present in the main control unit or the user interface. This much facilitates upgrading with improved exchangeable modules at a later point of time or to equip an already manufactured electrosurgical generator with an exchangeable module that is developed and brought to market much later. Due to the independent memory device in the data section, the extension module brings with it and provides to the electrosurgical generator all the data and information necessary for its operation. Owing to this "self-contained" memory device, this provides for a considerable boost in terms of an enlarged scope of future upgrades and further ease of upgrade.

This is in particular true if the exchangeable module is user exchangeable, e.g., in the case of an output socket. However, it also applies if the exchangeable module needs to be exchanged by a trained service technician, e.g., in the case of the high-voltage inverter unit. In either case, the new module automatically brings with it the data and information necessary for making proper use of the respective module and, if applicable, any enhanced functionality.

The invention is flexible concerning how the data transfer is to be performed between the exchangeable module and the main control unit as well as the user interface. This can be performed using a standard USB connection, a CAN interface which is particularly useful if CAN is already used for an internal data bus of the electrosurgical generator anyway, Ethernet or any other wired or wireless technology. Employing wireless technology may provide the advantage of allowing for a galvanically isolated data transfer.

In an advantageous embodiment, the control unit operates the inverter unit such as to supply the high-frequency voltage to the respective output socket module according to a mode retrieved from the data section of said output socket module. Accordingly, the output socket which is usually just at the receiving end will in this case provide data, namely a specific mode to be applied by the instrument plugged into the particular output socket mode, from its independent memory device to the main control unit in order to operate the inverter unit in a special way. Thereby, the output socket with its independent memory device actively influences operation of the electrosurgical generator. This considerably broadens the field of use and the flexibility in terms of additional modes to be provided, in particular by later designed output sockets and their respective electrosurgical instruments.

Advantageously, the user interface comprises a display manager being configured such as to show data and information concerning the respective module in accordance with the representation rules as stored in the data section of the respective module. The user interacts with the electrosurgical generator by the user interface with its display and gets the required information from the display. Further, in a modern user interface using a touchscreen device as a display, the user also enters his inputs thereby (although this is not a requirement and also a conventional key or button type input is possible). The type of data to be presented and the inputs possible vary between different electrosurgical instruments and different output sockets. New options, e.g., new modes, provided by the output socket and stored in its independent memory device, may require different kind of interaction with different kind of presentation of data and/or also different kind of input parameters. In order to take care of this, the independent memory device comprises data on respective representation rules, and upon reading out this data the display manager presents the data or information accordingly. With respect to electrosurgical generators having more than one output socket, it is beneficial if a plurality of output sockets can be seen at once by the user. In order to enable this, the display manager is preferably enabled of providing a compartmentalized display having two or more sections, each of the sections being assigned to one of the modules. Thereby, depending on the actual equipment of the electrosurgical generator, the presentation and the overview as given to the user can be adapted according to the modules which are actually installed.

Preferably, graphic representation in each of the sections is generated and displayed in a manner as defined by the representation rules stored on the respective assigned module, preferably employing actual data concerning the respective assigned module. Thereby, even for a greatly different modules, output sockets and inverter units, a specific and tailored representation can be provided on the user interface, in particular in the respective section belonging to the respective module.

Advantageously, the independent memory device comprises stored data blocks on haptic, acoustical and visual representation of data and status information concerning the respective module, preferably activation and error tones, graphical information, in particular structure of menus and/or color schemes to be displayed on the user interface. This allows for distinct and new information, thereby enabling the user to clearly differentiate which module, for example which output socket, is actually being actuated or which one is actually being subjected to an error condition. This allows addition of new functionalities that have not been foreseen by the time when the electrosurgical generator and its main control unit were developed and manufactured.

Preferably, the data blocks further comprise data sets on interaction, preferably relating to action to be performed on user interaction, in particular hand and/or pedal switch activation. Thereby, a new or altered types of required interaction by the user can be implemented and the corresponding data can be conveyed to the main control unit of the electrosurgical generator, thereby enabling proper control even of sophisticated electrosurgical instruments or modes.

Further preferably, the data blocks further comprise data sets on static information, preferably on-screen user manuals, and/or interactive information, preferably interactive user guides. This allows for a convenient and direct access to additional information that is necessary for proper operation of the respective module. This is a huge advantage not only for ease of access, but also for safety of use since by bringing along its own user manual it is ensured that the correct user manual or other respective information is used, namely that one which belongs to the specific module. Confusion or accidental reference to a different and non-applicable manual or other information is thereby effectively avoided. In an advantageous embodiment, the on-screen user manuals can be stored directly, e.g., as a pdf file or as hypertext file, or indirectly, e.g., by a link to a manual at a website. This allows for convenient access by the user. The latter option of the link to a website has further the advantage, that thereby access to the most recent, updated version can be guaranteed. Advantageously, the independent memory device further comprises stored data blocks on additional functionalities, e.g., modes and/or mode sequences. Preferably, the independent memory device further comprises stored data blocks on additional functions, restrictions, limitations depending on presence of other modules. Thereby the scope of available modes (sequences) and their functionality can be increased. An example for such mode sequences are predefined mode sequences, which are also called mode algorithms. Such a succession of different settings modes allows for performing of much more complicated tasks in a controlled stepwise manner.

Advantageously, the independent memory device has a writable section, comprising data fields for number of uses, usage history, last time of usage, cleaning cycles, instrument fitness state and/or module fitness state. This is particularly useful for user-exchangeable modules. Thereby usage status of the respective module can be controlled, even if this particular module is placed in different electrosurgical generators. This allows monitoring usage of this particular module, independent from the number of electrosurgical generators it was used in, and to provide an indication of maintenance or other service is needed.

Preferably, the data section further comprises a microcontroller configured to access the independent memory device and a communication interface for communication with the main control unit. Said microcontroller, which can be a socket module controller in the case of output socket, manages the local independent memory device and retrieves data needed for operation and handles communication to and from the main control unit. Advantageously, the microcontroller is configured to retrieve data sets on interaction from the independent memory device and to determine locally whether an interaction condition, e.g., handswitch actuation or instrument activation, is met and to communicate this to the main control unit. Further, special tasks in particular peculiar to new modes, mode sequences or other functionalities can be handled locally at the module by virtue of said microcontroller, e.g., monitoring new fault conditions that may occur due to any of the new modes, mode sequences or other functions; if so, then a general fault signal can be communicated to the main control unit. The local microcontroller thus facilitates handling of functions, in particular with respect to new modes, mode sequences or other functionality, and a new surgical instrument.

Further, in a preferred embodiment that may deserve independent protection, the microcontroller may be configured to manage operation of the inverter unit supplying high-frequency voltage to the respective output socket and further manages the content of the display of the user interface belonging to said output socket, in particular what is to be shown on the display and how the user can interact. In this scenario, the main control unit is reduced to just providing basic functionality including access to the user interface with the display. In the case of the display this could mean that compartmentalization of the display into several sections is handled by the user interface centrally, and populating the content of any of the sections is being controlled by the local microcontroller of the respective module (output socket). To this end the local microcontroller is preferably equipped with an interpreter for graphic language defining menu structure, active elements to be displayed, color schemes and stylesheets. This has the advantage that presentation of the data and acceptance of inputs, in particular in case of a touchscreen display, is handled by the microcontroller of the respective output socket module, wherein data sets about the special modes provided by this module are stored in the local independent memory device. The main control unit will thus be reduced to provide basic services, and allowing for general settings affecting the electrosurgical generator as a whole. Matters concerning the respective module (output socket) will be handled by the microcontroller of said module in cooperation with the local independent memory device. Thereby it is much facilitated to customize electrosurgical generators of different functionalities by equipping it with different modules (output sockets), and to modernize them by providing upgraded modules (output sockets). A similar rationale applies for modules having other functionalities, like being an inverter unit module etc.

Preferably, the power section of an inverter module forms the inverter unit of the electrosurgical generator, preferably a secondary inverter unit, e.g., an additional inverter for providing ultrasound HF voltage. Although much of the description hitherto has been provided with respect to the module being an output socket, it is also possible to have the module being configured as an (additional) inverter unit. Thereby, a second or third inverter unit can be provided for the electrosurgical generator. Further, thereby it is enabled to generate high-frequency voltage of different frequencies and kinds. This allows e.g., for true dual activation of different instruments, like two bipolar instruments. Further, this allows for generation of different kinds of HF frequency, e.g., in the radiofrequency range and in the ultrasound range. Thereby, also advanced instruments requiring such complex multi-frequency voltages supply can be employed.

Different kinds of power sections can be employed for the modules, in addition to output sockets of inverters. Advantageously, the power section forms a pump, a controlled drive for a motor-driven instrument, or a controlled gas supply, in particular for a gas supply of the electrosurgical instrument. Since the procedures and data necessary for driving these modules can be supplied by the independent memory device and they thus become independent from the original programming of the main control unit of the electrosurgical generator, an unprecedented variety of different modules can thus be used accordingly to the concept of the present invention.

The invention is explained in more detail below with reference to an advantageous exemplary embodiment. In the figures:
- Fig. 1: shows an electrosurgical generator according to an exemplary embodiment with an attached electrosurgical instrument;
- Fig. 2: shows a schematic functional diagram of the electrosurgical generator according to Fig. 1;
- Fig. 3: shows a schematic functional diagram of an output socket module;
- Fig. 4: show schematic signal and power flow diagrams for the output socket module of Fig. 3;
- Fig. 5A, B: show sample display content of the electrosurgical generator in different configurations;
- Fig. 6: show schematic signal and power flow diagrams for an inverter unit module; and
- Fig. 7: shows a schematic overview of modules in the electrosurgical generator.

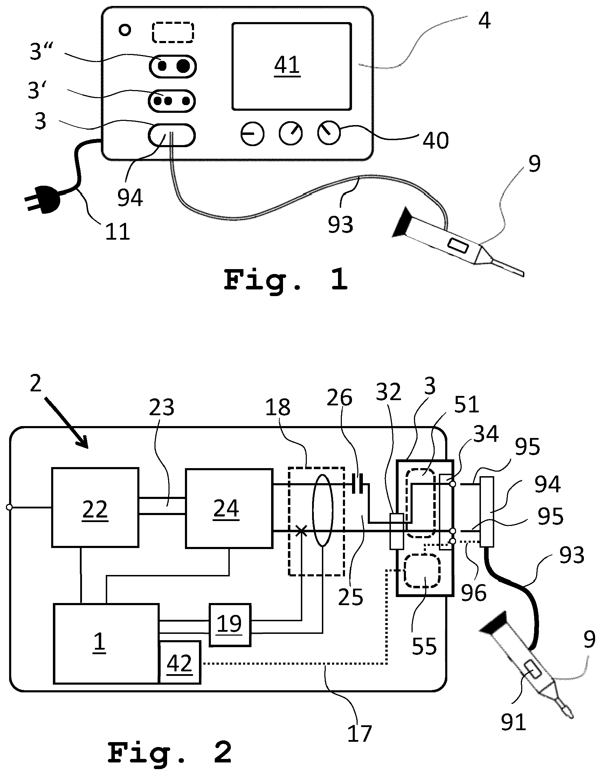

An electrosurgical generator according to an exemplary embodiment of the invention is illustrated in Fig. 1. The electrosurgical generator, comprises a housing having a user interface 4 and at least one output socket 3 (in the depicted exemplary embodiment a total of three output sockets 3, 3', 3") for connection of an electrosurgical instrument 9. A power supply cable 11 is provided which is connectable to an electrical power source which may be an electricity grid, like AC mains in a building, or an off-grid source of electric energy, like a 12 Volt or 24 Volt battery in a vehicle or mobile hospital. Further, the user interface 4 comprises an input device like knobs 40 for inputs by the user and a display 41 that may optionally be configured as a touch-screen thereby also doubling as the input device. The display 41 shows information concerning the inputs made by the user and the status of the electrosurgical generator. By virtue of the user interface 4 the user can issue directions and commands to a control unit 1 which controls operation of the electrosurgical generator and its components, including frequency and voltage of the AC voltage emitted by the output socket 3 as well as modes of operation.

Said electrosurgical instrument 9 comprises a cable 93 with a high voltage plug 94 which is to be plugged-in into one of the output sockets, in the depicted embodiment into output socket 3, in order to supply the high-frequency alternating voltage for operation of the electrosurgical instrument 9.

Fig. 2 shows a schematic functional diagram of an internal circuitry 2 of the electrosurgical generator. It comprises a power supply unit 22 that is fed by electrical energy by the supply cable 11, the power supply unit 22 feeding a DC bus 23 connected to an inverter 24 configured for generating high-frequency alternating current in a high-voltage range of a few kilovolts. Operation of the inverter 24 is governed by the control unit 1 which in turn is connected with the user interface 4 such that the user can issue directions and commands for operation of the electrosurgical generator.

The control unit 1 generates corresponding control signals and governs the relevant components of the internal circuitry 2 according to these instructions and commands. According to this, the inverter 24 generates high-frequency alternating voltage which is to be supplied to the electrosurgical instrument 9. To achieve this, the high-frequency high-voltage output emitted by the inverter 24 is routed via an output connection 25 to a power connector 32 at the output socket 3. The output connection 25 typically comprises two conductors, one for a neutral electrode NE and one other for an active electrode AE, which comprises a DC blocking capacitor 26 in its conductor. The electrosurgical instrument 9 with its cable 93 and attached plug 94 can be plugged into the output socket 3. Actual voltage and current of the high-frequency alternating voltage as being supplied to the output socket 3 are measured by a voltage/current sensor 18. Its measurement signals are fed back by a feedback unit 19 to the control unit 1 of the electrosurgical generator.

There may be various electrosurgical instruments 9 of different kinds being differently configured, e.g., unipolar or bipolar, and having different plugs 94 to be attached into one of a variety of different output socket 3, 3', 3". The plugs 94 may be differently configured, in particular they may have one or two prongs 95 for the high-frequency alternating voltage, depending on whether it is a unipolar or bipolar electrosurgical instrument 9. Further, an optional additional prong 96 may be provided for data signals, however data transmission to the electrosurgical instrument 9 may also be contactless.

There are various kinds of output sockets 3, 3' 3", an exemplary list comprising (but not being limited to) unipolar, bipolar and universal output sockets.

Each of the output sockets 3, 3', 3" is configured to be an exchangeable module 5. The module 5 comprises a power section 51 for the high-frequency voltage used to drive the electrosurgical instrument 9, and a data section 55. The power section 51 comprises a power line set 33 for routing the power of from the connector 32 to a front side plug socket 34 which is configured as a receptacle for the plug 94 of the electrosurgical instrument 9.

The data section 55 further comprises an independent memory device 6 and an optional microcontroller 7. The independent memory devices 6 comprises data blocks having stored data for operating the module 5 in its capacity as an output socket 3 in the electrosurgical generator. The data and information stored in the independent memory device 6 can be accessed by the electrosurgical generator, in particular its main control unit 1. To this end, the module 5 comprises a communication interface 50 which can exemplary be configured as being as a USB interface, a CAN interface or an Ethernet interface. The communication interface may also be configured as a wireless interface 50', e.g., for Bluetooth wireless connection. Thereby, the main control unit 1 can retrieve the information and data from the independent memory devices 6 that is necessary for proper usage of the respective module 5.

The independent memory device comprises data relating to the operation of the respective module 5 and to representation rules defining how operational data of the respective module 5 is to be displayed on the user interface for, in particular its display 41. To this end, the independent memory device 6 comprises data blocks 61 on specific functionalities, like modes to be performed by the electrosurgical generator and its inverter unit 24 upon using the electrosurgical instrument 9 connected to the respective output socket 3, and on additional functionalities e.g., mode sequences which are a succession of different mode settings.

Further, the independent memory device 6 comprises data blocks 62 on visual representation of data and status information regarding the respective module 5 and the instrument and 9 connected thereto. This comprises in particular graphical information how to show the data and information concerning the respective module 5 and the instrument 9 on the screen of the user interface 4 as well as activation tones to be provided to the user by the user interface 4. However, this may also comprises other presentation data, in particular with respect which color shall be used by a multicolor LED 36 to illuminate the corresponding plug socket 34 by means of a surrounding light guide 35. This is an important safety feature as thereby the user is clearly notified by the illumination which output socket 3 is activated and which mode is selected for the instrument, like blue for bipolar, yellow for monopolar or purple for a special mode (e.g., for a thunderbeat instrument).

Yet further, the independent memory device 6 may comprise data blocks comprising data sets 63 on interactions, preferably relating to interactions like the user activating a handswitch 91 of the electrosurgical instrument 9 and/or pedal switch (not shown) of the electrosurgical generator. Yet further, there may be comprised data sets 64 concerning static information, in particular an on-screen user manual, or an interactive user guide. Further, the independent memory device 6 may comprise data blocks 65 on additional functions or restrictions which may be conditionally, in particular depending on the presence or status of other devices of the electrosurgical generator. For example, additional functionalities may be activated if the presence of a second inverter unit was detected. In addition, the independent memory device 6 features a writable section 67 that comprises data fields concerning usage history, number of uses, last time of usage, cleaning cycles and fitness date of the respective module 5 and/or the attached electrosurgical instrument 9.

By providing the optional microcontroller 7 the range of functionality of the module 5 can be extended. The microcontroller 7 interacts with the independent memory device 6 and retrieves data blocks and data sets therefrom. Thereby, it is possible to perform the handling of functions locally on the respective module 5. Apart from rather simple tasks like detecting activation of the handswitch 91 on the instrument 9 in combination with an instrument interface 56, this may include more complex tasks by executing certain modes or mode sequences in conjunction with the inverter unit 24 via a communication interface 50 on the respective module 5. Importantly, the microcontroller 7 may also control - dependent on the mode selected and retrieved from the independent memory device 6 or on the a result of communication with the main control unit 1 - if and when the high-frequency voltage shall be allowed to be conveyed to the electrosurgical instrument 9 by activation of an protection relay 53. This is an important aspect that allows for improved safety features, in particular in combination with rather complex modes and mode sequences.

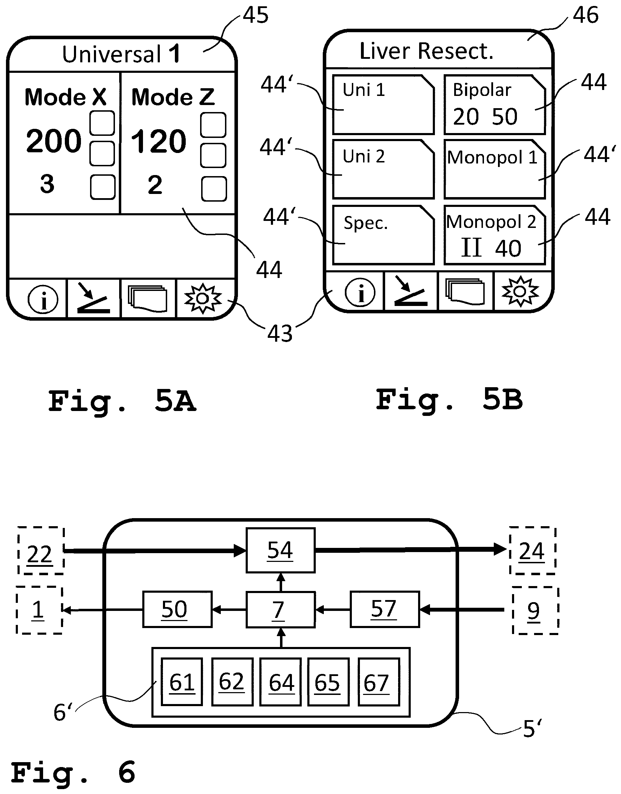

Concerning displaying according to the representation rules as they are stored in the independent memory devices 6, reference is made to Fig. 5A and 5B. The user interface 4 comprises a display manager 42 (see Fig. 2) that is configured to show data and information concerning operation of the electrosurgical generator and its components on the display 41. Concerning the output socket 3 being configured as module 5 and comprising representation rules in its independent memory device 6 defining how operational data of said module 5 is to be displayed, an example is shown in Fig. 5A. In the lowermost portion system information 43 concerning the electrosurgical generator as a whole is represented. In the shown example, there are four different fields for accessing further information, for configuring a pedal switch (or handswitch), for calling procedures and for general settings. This is generally controlled centrally by the user interface 4 of the electrosurgical generator. The module 5, in this case the output socket 3 concerned "Universal 1", is shown in the top section 45 of the display 41. However, for the graphical representation of data of the module 5, the display manager 42 provides one or more sections 44.

The content to be displayed in the respective section 44 is defined by the representation rules as they are stored in the data blocks of the independent memory device 6 of the concerned module 5. Thereby it is governed which kind of graphic user interface shall be used, which audio signals, which are the modes to be used, and corresponding reference documentation, in particular the respective manual, will be provided. In the depicted embodiment, two modes "X" and "Z" are provided, one for cutting and one for sealing, respectively. As per the representation rules stored in the independent memory device 6, indications of energy level ("200" and "120") are provided, and further indicators for selected effects ("3" and "2"). Further, as per the representation rules for the GUI stored in the independent memory device 6, click buttons are provided with respect to either mode, in the depicted embodiment three click buttons (to the right of energy level indications). - It is to be noted that the presentation given in section 44 could be completely different for another output socket 3 (or for another module 5 generally) since its content is merely defined by the graphical representation rules which are stored in the independent memory device 6 of the respective module 5.

In Fig. 5B a variant is shown relating to an electrosurgical generator which comprises six output sockets 3 being configured as a module 5 having an independent memory device 6. The display manager 42 provides for either of them a section 44 on the display 41, wherein as depicted in Fig. 5B sections 44' are for those output sockets 3 which are not actively used at that time. Actively used are the output sockets 3 for "Bipolar" and for "Monopol 2", and their graphic representation in the respective section 44 is in either case defined by the respective independent memory device 6, as explained above. By selecting one of the sections 44, the particular output socket 3 will be put in front and a graphic representation similar to that of Fig. 5A will be given as governed by the independent memory device 6 and the optional microcontroller 7 of the respective module 5.

While the foregoing applies to an output socket 3 being configured as module 5, it is further possible to configure other components of the electrosurgical generator as a module, too. For example, the inverter unit 24 can be configured as a module 5'.

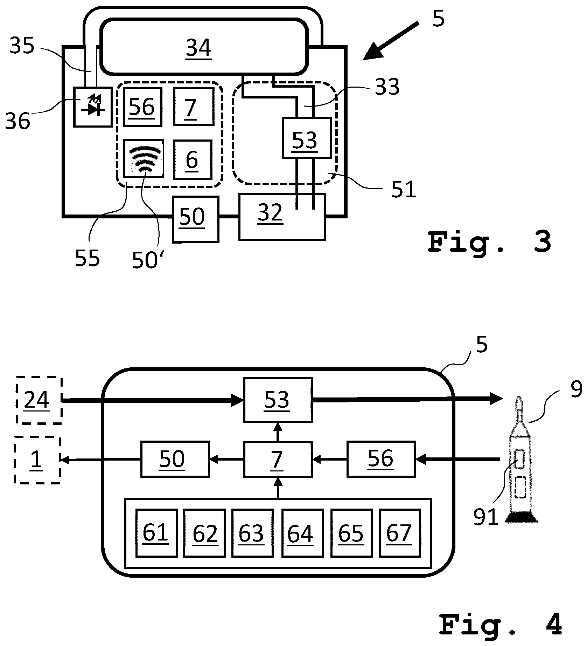

This is shown in Fig. 6 and will be briefly explained in the following. The general principle follows that of the output socket 3 as a module 5 shown in Fig. 4. Elements having the same or like function will have the same reference numeral. The module 5' will also feature an independent memory device 6' which may, however, be configured differently, in the present example for operating as an inverter unit 24. To this end, the independent memory devices 6' comprises data blocks 61 on specific functionalities, like modes that are to be performed upon using the electrosurgical instrument 9 connected to the respective output socket 3 to which the high-frequency voltage to be generated by the inverter unit 24 is supplied. Further comprised are data blocks 62 on visual representation of data and status information regarding the inverter unit 24 on the display 41 as well as activation tones to be provided to the user by the user interface 4. Static information, like those for an on-screen user manual or interactive user guide, is stored in data blocks 64, and data on additional functions or restrictions, in particular with respect to certain output sockets or instruments 9 plugged-in, is comprised in data blocks 65. Finally, a writable section 67 is provided that comprises data fields concerning usage history, number of uses, total amount of energy generated and other fitness data of the inverter unit 24. In accordance with this and in consideration of communication with the main control unit 1 via the communication interface 50, the microcontroller 7 controls operation of the inverter unit 24 by energizing an activation relay 54 upon detection of the instrument 9 being activated by means of an activation detector 57 configured to detect activation of the instrument 9.

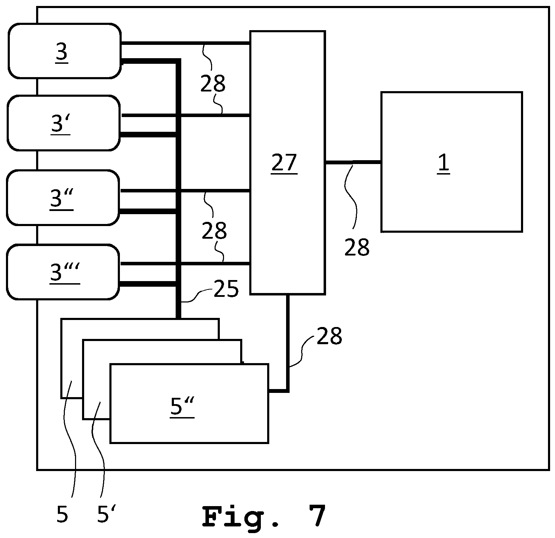

An overview of an embodiment of the electrosurgical generator comprising several modules of different kinds is shown in Fig. 7. The electrosurgical generator comprises a plurality (in the depicted embodiment four) output sockets 3 configured as modules 5 and the inverter 24 being configured as module 5', which are connected by the output connection 25. The modules and the main control unit 1 are being communicatively connected by means of a communication hub 27. Further, additional modules may be provided, like a module 5" for a controlled gas supply, in particular of a noble gas like Argon to the site of cutting/sealing (or for a controlled drive of a motor-driven instrument).

The main control unit 1 communicates with the various modules via the communication hub 27. Communication lines between the communication hub 27, the main control unit 1 and various modules 3, 5 can be made directly or via a bus 28, like USB, or via network, like Ethernet or CAN.

By virtue of such a configuration, different types of electrosurgical generators can be built using the modules as standardized building blocks having the same interface. This allows for rapid and efficient manufacturing even of a high variety of different electrosurgical generators.

## Claims

1. Electrosurgical generator configured to output a high-frequency alternating voltage to an electrosurgical instrument (9), comprising a power supply unit (22), an inverter unit (24) generating a high-frequency voltage which is output to at least one output socket (3) for connection of the electrosurgical instrument (9), a main control unit (1) configured to control operation of the electrosurgical generator, and a user interface (4) for user input/output being functionally connected to the control unit (1),
**characterized in that**
the inverter unit (24) and/or the output socket (3) is configured as exchangeable module, wherein the module (5) comprises
- a power section (51) for the high-frequency voltage,
and
- a data section (55) comprising an independent memory device (6) having stored data sets on
(i) modes relating to usage of the respective module, and
(ii) representation rules defining how operational data of the respective module is to be displayed on the user interface, wherein the independent memory device is operationally connected to the control unit.

2. Electrosurgical generator of claim 1, wherein at least the module (5) for the output socket (3) is exchangeable by the user.

3. Electrosurgical generator of claim 1 or 2, wherein the control unit (1) operates the inverter unit (24) such as to supply the high-frequency voltage to the respective output socket (3) according to a mode retrieved from the data section (55) thereof.

4. Electrosurgical generator of any of the preceding claims, wherein the user interface (4) comprises a display manager (42) being configured such as to show data and information concerning the respective module in accordance with the representation rules as stored in the data section of the respective module.

5. Electrosurgical generator of the preceding claim, wherein the display manager (42) is enabled of providing a compartmentalized display having two or more sections (44), each of the sections (44) being assigned to one of the modules (5).

6. Electrosurgical generator of the preceding claim, wherein graphic representation in each of the sections (44) is generated and displayed in a manner as defined by the representation rules stored on the respective assigned module, preferably employing actual data concerning the respective assigned module.

7. Electrosurgical generator of any of the preceding claims, wherein the independent memory device (6) comprises stored data blocks (62) on haptic, acoustical and visual representation of data and status information concerning the respective module, preferably activation and error tones, graphical information, in particular structure of menus and/or color schemes to be displayed on the user interface.

8. Electrosurgical generator of the preceding claim, wherein the data blocks further comprise data sets (63) on interaction, preferably relating to action to be performed on user interaction, in particular hand and/or pedal switch activation.

9. Electrosurgical generator of claim 7 or 8, wherein the data blocks further comprise data sets (64) on static information, preferably on-screen user manuals, and/or interactive information, preferably interactive user guides.

10. Electrosurgical generator of any of claims 7 to 9,
wherein the independent memory device further comprises stored data blocks (61) on additional functionalities, preferably modes and/or mode sequences.

11. Electrosurgical generator of any of claims 7 to 10,
wherein the independent memory device further comprises stored data blocks (65) on additional functions, restrictions, limitations depending on presence of other modules.

12. Electrosurgical generator of any of the preceding claims, wherein the independent memory device has a writable section (67), comprising data fields for number of uses, usage history, last time of usage, cleaning cycles, instrument fitness state and/or module fitness state.

13. Electrosurgical generator of any of the preceding claims, wherein the data section further comprises a microcontroller (7) configured to access the independent memory device (6) and a communication interface (50) for communication with the main control unit (1).

14. Electrosurgical generator of the preceding claim, wherein the microcontroller (7) is configured to retrieve data sets on interaction from the independent memory device (6) and to determine locally whether an interaction condition, preferably handswitch actuation or instrument activation, is met and to communicate this to the main control unit (1).

15. Electrosurgical generator of any of the preceding claims, wherein the power section (51) of an inverter module (5') forms the inverter unit (24) of the electrosurgical generator, preferably a secondary inverter unit, e.g., an additional inverter for providing ultrasound HF voltage.

16. Electrosurgical generator of any of the preceding claims, wherein the power section (51) forms a pump, a controlled drive for a motor-driven instrument, or a controlled gas supply, in particular for a gas supply of the electrosurgical instrument (9).
